(19) Europäisches Patentamt
European Patent Office
Office européen des brevets

(11) **EP 0 882 444 B1**

(12) **EUROPÄISCHE PATENTSCHRIFT**

(45) Veröffentlichungstag und Bekanntmachung des Hinweises auf die Patenterteilung:
**28.09.2005   Patentblatt 2005/39**

(51) Int Cl.$^7$: **A61K 7/135**

(21) Anmeldenummer: **98105084.2**

(22) Anmeldetag: **20.03.1998**

(54) **Mittel zum Bleichen von Haaren**

Hair bleaching composition

Composition pour la décoloration des cheveux

(84) Benannte Vertragsstaaten:
**DE ES FR GB IT**

(30) Priorität: **05.06.1997   DE 19723538**

(43) Veröffentlichungstag der Anmeldung:
**09.12.1998   Patentblatt 1998/50**

(73) Patentinhaber: **Wella Aktiengesellschaft**
**64274 Darmstadt (DE)**

(72) Erfinder:
• **Schmitt, Manfred**
  **64646 Heppenheim (DE)**
• **Balzer, Wolfgang R.**
  **64665 Alsbach-Hähnlein (DE)**
• **Lenz, Uwe**
  **64673 Zwingenberg (DE)**

(56) Entgegenhaltungen:
**EP-A- 0 574 696        US-A- 5 888 484**

**Beschreibung**

Gegenstand der vorliegenden Erfindung ist ein aus zwei Komponenten erhältliches Mittel zum Entfärben oder Blondieren von Haaren, insbesondere menschlichen Haaren.

Zum Entfärben oder Blondieren von Haaren werden üblicherweise oxidierende Zubereitungen verwendet, welche durch Auflösen eines sogenannten Blondierpulvers (Pulvergemisch aus Alkalisalzen und anorganischen Persalzen, wie zum Beispiel Natrium- oder Ammoniumpersulfat) in einer wäßrigen Wasserstoffperoxidlösung erhalten werden.

Die Verwendung derartiger Blondierpulver, welche notwendigerweise aus mehreren Bestandteilen zusammengesetzt sind, bringt jedoch eine Vielzahl von Nachteilen mit sich. So kommt es durch die Verwendung von Rohstoffen mit unterschiedlicher Dichte häufig während des Transportes oder der Lagerung zur Trennung der unterschiedlichen Pulverbestandteile, wobei sich die schweren Pulverbestandteile im unteren Teil und die leichteren Pulverbestandteile im oberen Teil des Behältnisses ansammeln. Diese Entmischung hat zur Folge, daß die gleiche Pulvermenge je nach ihrem Entnahmeort eine unterschiedliche chemische Zusammensetzung und somit auch eine unterschiedliche Blondierwirkung aufweisen kann.

Um dieser Entmischung entgegenzuwirken, ist es erforderlich, vor jeder Pulverentnahme das Pulver gründlich zu schütteln, was der Verwender in der Regel jedoch nicht tut.

Eine Entmischung kann auch durch den Einsatz von Pulvergemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit der Wasserstoffperoxidlösung - zu einer starken Staubentwicklung neigen, was zu einer Reizung der Atmungsorgane führen kann. Weiterhin besitzen derartige Pulvergemische aufgrund der geringen Korngröße eine große Oberfläche, wodurch eine Aufnahme von Feuchigkeit beim Öffnen und Schließen des Behältnisses und damit eine Verringerung der Blondierwirkung infolge der Desaktivierung des Sauerstoffträgers begünstigt wird.

Die Zubereitung der gebrauchsfertigen Mischung erfolgt zum einen durch Verrühren der Bestandteile in einer Schale, zum anderen durch Vermischen in einer Anschüttelflasche, wobei insbesondere das Anschütteln oftmals mit einer lästigen Staubentwicklung beim Einfüllen der Komponenten in die Schüttelflasche verbunden ist.

Es wurden bereits zahlreiche Versuche unternommen, dieses Problem zu lösen.

So wird in der DE-OS 40 26 235 vorgeschlagen, anstelle eines Blondierpulvers eine Mischung, bestehend aus einem Granulat der anorganischen Persulfate und einem Granulat der übrigen Bestandteile des Blondiermittels, zu verwenden. Hierdurch kann zwar das Problem der Staubentwicklung behoben werden, das Problem der Entmischung kann auf diesem Wege jedoch nicht gelöst werden, da es technisch äußerst schwierig ist, Einzelgranulate mit identischer und konstanter Korngröße oder Schüttgewicht herzustellen. Weiterhin kann es aufgrund der unterschiedlichen Löslichkeit der einzelnen Granulate zu einer Beeinträchtigung der Blondierwirkung kommen. Es erscheint zudem auch aus ökonomischen Gesichtspunkten wenig sinnvoll, anstelle eines einzelnen Granulates eine Mischung aus mehreren Granulaten herzustellen.

In der DE-AS 20 23 922 wird empfohlen, ein Granulat anstelle des Pulvers zu verwenden. Dieses Granulat soll durch Besprühen des alle erforderlichen Bestandteile enthaltenden Blondierpulvers mit einer wäßrigen, alkoholischen oder wäßrig-alkoholischen Polymer-Lösung in einem geeigneten Mischer hergestellt werden.

Bei diesem Granulierverfahren kommt es jedoch zu einem starken Ammoniakverlust, wodurch die Blondierwirkung des Granulats verschlechtert wird. Dieser Ammoniakverlust soll durch die Erhöhung des Ammoniumsalzanteils in dem eingesetzten Pulver bzw. durch den Zusatz von Ammoniak zu der Polymerlösung kompensiert werden. Da der verfahrensbedingte Ammoniakverlust bei diesem Granulationsverfahren stark schwankt, ist es mit diesem Verfahren nicht möglich, ein Granulat mit einer konstanten chemischen Zusammensetzung herzustellen.

Weiterhin kann auch bei diesen Mitteln die Staubfreiheit nicht völlig gewährleistet werden, da durch Reibung der Granulatteilchen aneinander, zum Beispiel während des Transportes, Feinstaub gebildet wird.

Eine Verwendung solcher Blondiergranulate in einer Anschüttelflasche ist wegen der schlechten Löslichkeit der Granulate nicht möglich.

In der EP-PS 0 560 088 wird ein pulverförmiges Mittel zum Blondieren von Haaren beschrieben, bei dem zur Verhinderung der Staubbildung ein Öl oder flüssiges Wachs zugesetzt wird. Aber auch hierdurch kann eine völlige Staubfreiheit nicht erreicht werden. Zudem ergibt sich durch den Wassergehalt der eingesetzten pulverförmigen Rohstoffe und die kompakte Pulverform eine Desaktivierung der Sauerstoffträger, wodurch das Produkt instabil wird und seine Bleichwirkung verliert.

Weiterhin sind derartige Blondiermittel aufgrund ihres spezifischen Gewichtes und ihres hydrophoben Charakters für die Verwendung in der Auftrageflasche ungeeignet, da das Pulver in der Wasserstoffperoxidlösung nach unten sinkt und nicht ausreichend benetzt wird, wodurch eine inhomogene Mischung mit einem hohen Anteil an ungelöstem Pulver erhalten wird, durch die die Austrittsdüse der Auftrageflasche verstopft wird. Der Zusatz von Tensiden zur Verbesserung der Löslichkeit des Pulvers ist ebenfalls problematisch, da hierdurch die Lagerfähigkeit des Pulvers beeinträchtigt wird.

In der DE-PS 38 14 356 werden breiartige Zweikomponenten-Zubereitungen zur Anfertigung einer auftragefähigen, breiartigen Zubereitung zum Bleichen von Humanhaaren beschrieben, welche als geeignete Verdickungsmittel Kieselsäure, speziell behandelte Tone und Silikate, hydriertes Rizinusölderivat und Paraffin- oder Montanwachse enthalten können. Diese Mittel haben jedoch den Nachteil, daß die verdickende Wirkung der verwendeten Verdicker nur unzureichend ist. So führt eine 1 : 1 Anwendung der pastenförmigen Komponenete 1 mit der Wasserstoffperoxidlösung schon zu niedrigviskosen Blondiermitteln, die bereits während der Einwirkzeit von den Haaren heruntertropfen. Anwendungen mit höheren Anteilen an Wasserstoffperoxidlösung sind noch dünner und können nicht benutzt werden, da sie schon während des Auftragens der Masse auf die Haare herunterlaufen.

Es bestand daher die Aufgabe, ein lagerstabiles pastenförmiges Mittel zum Entfärben oder Blondieren von Humanhaaren zur Verfügung zu stellen, welches vor Gebrauch durch einfaches Schütteln oder Anrühren mit einer flüssigen Wasserstoffperoxidlösung oder einer wasserstoffperoxidhaltigen Öl-in-Wasser-Emulsion vermischt wird und neben seiner absolut staubfreien Darreichungs- und Anwendungsform eine sehr gute Blondierwirkung bei gleichzeitig hervorragender Lagerstabilität gewährleistet.

Überraschend wurde nunmehr gefunden, daß die vorstehend beschriebenen Nachteile vermieden werden können, wenn man ein Blondiermittel in Form einer auf einer speziellen Kombination von Inhaltsstoffen basierenden Suspension oder Dispersion verwendet.

Gegenstand der vorliegenden Erfindung ist daher ein Mittel zum Entfärben oder Blondieren von Haaren, welches unmittelbar vor der Anwendung durch Vermischen einer cremeförmigen Blondiermittelsuspension mit einem Oxidationsmittel hergestellt wird und dadurch gekennzeichnet ist, daß die Blondiermittelsuspension eine Mischung aus (a) mindestens einem anorganischen Persalz, (b) mindestens einem alkalisch reagierenden Salz, (c) einer Verdickerkombination, bestehend aus einem Acrylsäurepolymer und mindestens einem Polymer aus der Gruppe der Cellulosen, Alginate und Polysaccharide, (d) mindestens einem Mineralöl, (e) mindestens einem flüssigen langkettigen hydrophoben Fettsäureester, (f) mindestens einem wachsförmigen langkettigen hydrophoben Fettsäureester und/oder synthetischen Bienenwachsersatzstoff, sowie (g) gegebenenfalls Hilfsstoffen und Zusatzstoffen darstellt.

Als anorganisches Persalz werden vorzugsweise Persulfate, beispielsweise Natriumpersulfat, Kaliumpersulfat, Ammoniumpersulfat oder Mischungen dieser Persulfate, eingesetzt, wobei die Persulfate in der Blondiermittelsuspension in einer Gesamtmenge von vorzugsweise 30 bis 65 Gewichtsprozent, insbesondere 35 bis 55 Gewichtsprozent, enthalten sind.

Als alkalisch reagierendes Salz werden vorzugsweise in wäßriger Lösung alkalisch reagierende Alkalimetallsalze oder Erdalkalimetallsalze, beispielsweise Natriumcarbonat, Natriumhydrogencarbonat, Magnesiumcarbonat, Ammoniumcarbonat, Ammoniumhydrogencarbonat, Natriumsilikate oder Mischungen dieser Salze, verwendet, wobei diese Salze in der Blondiermittelsuspension in einer Gesamtmenge von vorzugsweise 15 bis 45 Gewichtsprozent, insbesondere 18 bis 35 Gewichtsprozent, enthalten sind.

Als Polymer aus der Gruppe der Cellulosen, Alginate und Polysaccharide werden vorzugsweise Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum, alleine oder in Kombination miteinander, verwendet, wobei die Verwendung von anquellverzögernden Methylhydroxyethylcellulosen oder einer Kombination von Natriumalginaten mit Polysacchariden oder/und Cellulosen besonders bevorzugt ist.

Als besonders vorteilhaft hat sich hierbei die Kombination von Natriumalginaten mit Xanthan Gum sowie die Kombination von Natriumalginaten mit Methylhydroxyethylcellulose in einem Verhältnis von 1 zu 3 bis 3 zu 1, insbesondere von 1 zu 2 bis 2 zu 1, erwiesen.

Bezogen auf die Gesamtmenge der Blondiermittelsuspension werden die Cellulosen in einer Menge von 0,1 bis 20 Gewichtsprozent, vorzugsweise 0,2 bis 15 Gewichtsprozent, eingesetzt, wobei eine Einsatzmenge von 0,5 bis 12 Gewichtsprozent besonders bevorzugt ist.

Die Einsatzmenge für die Alginate und Polysaccharide beträgt, bezogen auf die Gesamtmenge der Blondiermittelsuspension, jeweils 0,1 bis 15 Gewichtsprozent, vorzugsweise 0,2 bis 12 Gewichtsprozent und insbesondere 0,5 bis 10 Gewichtsprozent.

Als Acrylsäurepolymer werden vorzugsweise hochmolekulare Acrylsäurepolymere mit einem Molekulargewicht von etwa 1.250.000 bis 4.000.000, beispielsweise die Handelsprodukte Carbopol® 940, Carbopol® 941, Carbopol® 954 und Carbopol® 981 der Firma BF Goodrich/USA oder die Handesprodukte Acrisint® 410, Synthalen® L und Synthalen® K der Firma 3V-Sigma/USA, eingesetzt.

Das Acrylsäurepolymer wird, bezogen auf die Gesamtmenge der Blondiermittelsuspension, vorzugsweise in einer Menge von 0,1 bis 3 Gewichtsprozent, insbesondere 0,1 bis 2 Gewichtsprozent, eingesetzt.

Die Verdickerkombination der Komponente (c) ist in der Blondiermittelsuspension in einer Gesamtmenge von etwa 0,5 bis 20 Gewichtsprozent, vorzugsweise 1,5 bis 17 Gewichtsprozent, enthalten.

Als Mineralöle eignen sich insbesondere flüssige Paraffine, beispielsweise Paraffin Perliquidum und Paraffin Subliquidum. Als flüssige, langkettige, hydrophobe Fettsäureester können beispielsweise Octylpalmitat, Isocetylpalmitat und insbesondere Isopropylpalmitat verwendet werden. Als wachsförmige, langkettige, hydrophobe Fettsäureester und/oder synthetische Bienenwachsersatzstoffe können synthetisches Bienenwachs (beispielsweise Lipowachs 6138G® der Firma Lipo Chemicals/USA), C18- bis C36-Fettsäuren (beispielsweise Synchrowachs AW1C der Firma Croda Chemicals Ltd./Großbritannien), hydrierte Kokosfettsäureglyceride (zum Beispiel Softisan® 100 der Firma Hüls AG/BRD), Glyceryltribehenat (beispielsweise Synchrowachs® HRC der Firma Croda Chemicals Ltd./Großbritannien), Fettsäureestergemische (beispielsweise Cutina BW® der Firma Henkel KGaA/BRD) und insbesondere Bienenwachs verwendet werden.

Besonders bevorzugt ist hierbei die Verwendung einer Kombination aus Bienenwachs, Isopropylpalmitat und Paraffin Perliquidum und/oder Paraffin Subliquidum.

Die Gesamtmenge an flüssigen langkettigen, hydrophoben Fettsäureestern beträgt, bezogen auf die Gesamtmenge der Blondiermittelsuspension, 0,5 bis 34 Gewichtsprozent, vorzugsweise 3 bis 26,5 Gewichtsprozent. Die wachsförmigen, langkettigen, hydrophoben Fettsäureester und/oder synthetischen Bienenwachsersatzstoffe werden, bezogen auf die Gesamtmenge der Blondiermittelsuspension, vorzugsweise in einer Gesamtmenge von 0,5 bis 10 Gewichtsprozent, insbesondere 0,5 bis 8 Gewichtsprozent, eingesetzt. Die Gesamtmenge an Mineralölen beträgt, bezogen auf die Gesamtmenge der Blondiermittelsuspension 3 bis 34 Gewichtsprozent, vorzugsweise 5 bis 25,5 Gewichtsprozent, wobei die Gesamtmenge der in der Blondiermittelsuspension enthaltenen Verbindungen der Komponenten (d), (e) und (f) etwa 20 bis 35 Gewichtsprozent, vorzugsweise 25 bis 29 Gewichtsprozent, beträgt.

Zusätzlich kann die cremeförmige Blondiermittelsuspension weitere für derartige Zubereitungen übliche Zusatzstoffe, wie zum Beispiel Siliciumdioxid, Titandioxid, Chelatisierungsmittel für Schwermetallionen, beispielsweise Ethylendiaminotetraessigsäure, Anfärbestoffe, beispielsweise Ultramarinfarbstoffe oder saure Farbstoffe, oder Parfüme, enthalten, wobei diese Zusatzstoffe in den für solche Mittel üblichen Einsatzmengen, beispielsweise das Siliciumdioxid und das Chelatisierungsmittel jeweils in einer Menge von 0,1 bis 3 Gewichtsprozent, und die Anfärbestoffe und Parfüme jeweils in einer Menge von 0,01 bis 1 Gewichtsprozent, eingesetzt werden.

Vorzugsweise enthält das erfindungsgemäße Blondiermittel keine Tenside und ist wasserfrei. Ein Wassergehalt von maximal bis zu 2,5 Gewichtsprozent ist jedoch zulässig.

Vor der Anwendung wird die cremeförmige Blondiermittelsuspension mit einem Oxidationsmittel, vorzugsweise einer wäßrigen Wasserstoffperoxidlösung oder wasserstoffperoxidhaltigen Öl-in-Wasser-Emulsion, zu einem auftragefähigen Blondierbrei vermischt, wobei dieses Vermischen in einer Schale oder durch Anschütteln in einer Auftrageflasche erfolgen kann.

Das Mischungsverhältnis von Blondiermittelsuspension zu Oxidationsmittel beträgt bei Verwendung einer 6- bis 12-prozentigen Wasserstoffperoxidlösung oder Wasserstoffperoxidemulsion 1 zu 1 bis 1 zu 3.

Das so erhaltene gebrauchsfertige Mittel zum Entfärben oder Blondieren von Haaren wird auf das Haar gleichmäßig aufgetragen und nach einer Einwirkungszeit von 15 bis 60 Minuten bei Raumtemperatur (20-25°C) beziehungsweise von 10 bis 50 Minuten bei Wäremeinwirkung (30-50°C) mit Wasser ausgespült.

Die cremeförmige Blondiermittelsuspension kann je nach ihrer Viskosität in Tuben oder Tiegel abgefüllt werden und ist äußerst leicht mit dem Oxidationsmittel vermischbar, was aus der leichten Anschüttelbarkeit und der kurzen Anmischzeit von unter 20 Sekunden für 100 g der gebrauchsfertigen Zubereitung ersichtlich ist. Neben der anwendungsfreundlichen Produktviskosität und der leichten Anmischbarkeit zeichnet sich das erfindungsgemäße Mittel durch eine hervorragende Auftragefähigkeit, Verteilbarkeit und Haftung am Haar sowie eine sehr hohe Blondierwirkung und ein breites Anwendungsspektrum aus. Weiterhin besitzt das erfindungsgemäße Mittel eine hervorragende Lagerstabilität über einen weiten Temperaturbereich, insbesondere eine sehr gute Kältestabilität (auch bei Temperaturen unterhalb von +10 °C).

Die nachfolgenden Beispiele sollen den Gegenstand näher erläutern, ohne diesen hierauf zu beschränken.

**Beispiele**

| Beispiel 1: Cremeförmige Blondiermittelsuspension | |
|---|---|
| 25,0 g | Kaliumpersulfat |
| 18,0 g | Ammoniumpersulfat |
| 23,0 g | Natriummetasilikat |
| 2,0 g | Natriumalginat |
| 2,0 g | Xanthan Gum |
| 0,5 g | Acrylsäurepolymer (CTFA-Carbomer) |

(fortgesetzt)

| **Beispiel 1:** Cremeförmige Blondiermittelsuspension | |
|---|---|
| 5,0 g | Isopropylpalmitat |
| 23,5 g | Paraffin Perliquidum |
| 0,5 g | Bienenwachs |
| 0,4 g | Ethylendiaminotetraessigsäure |
| 0,1 g | Ultramarinblau |
| 100,0 g | |

| **Beispiel 2:** Cremeförmige Blondiermittelsuspension | |
|---|---|
| 25,0 g | Ammoniumpersulfat |
| 18,0 g | Natriumpersulfat |
| 23,0 g | Natriummetasilikat |
| 2,5 g | Natriumalginat |
| 1,5 g | Xanthan Gum |
| 0,5 g | Acrylsäurepolymer (CTFA-Carbomer) |
| 11,0 g | Isopropylpalmitat |
| 17,5 g | Paraffin Perliquidum |
| 0,5 g | Bienenwachs |
| 0,5 g | Ethylendiaminotetraessigsäure |
| 100,0 g | |

| **Beispiel 3:** Cremeförmige Blondiermittelsuspension | |
|---|---|
| 25,0 g | Ammoniumpersulfat |
| 18,0 g | Kaliumpersulfat |
| 23,0 g | Natriummetasilikat |
| 1,5 g | Natriumalginat |
| 2,5 g | Xanthan Gum |
| 1,0 g | Acrylsäurepolymer (CTFA-Carbomer) |
| 15,0 g | Isopropylpalmitat |
| 12,0 g | Paraffin Perliquidum |
| 1,5 g | Bienenwachs |
| 0,5 g | Ethylendiaminotetraessigsäure |
| 100,0 g | |

| **Beispiel 4:** Cremeförmige Blondiermittelsuspension | |
|---|---|
| 25,5 g | Ammoniumpersulfat |
| 18,0 g | Kaliumpersulfat |
| 23,0 g | Natriummetasilikat |
| 1,5 g | Natriumalginat |
| 2,5 g | Xanthan Gum |
| 0,5 g | Acrylsäurepolymer (CTFA-Carbomer) |
| 15,0 g | Isopropylpalmitat |
| 12,0 g | Paraffin Perliquidum |
| 0,5 g | Bienenwachs |
| 0,5 g | Ethylendiaminotetraessigsäure |

(fortgesetzt)

| Beispiel 4: Cremeförmige Blondiermittelsuspension | |
|---|---|
| 1,0 g | Siliciumdioxid |
| 100,0 g | |

**Herstellung der Blondiermittelsuspension im Technikummaßstab (10 kg Ansatz)**

Das Isopropylpalmitat, das Parafifin Perliquidum und das Bienenwachs werden in einem Unimix-Rührwerk (Type SR 15) vorgelegt und bei etwa 54° C geschmolzen und sodann unter Rühren auf etwa 30° C abgekühlt. Die übrigen Bestandteile werden zunächst in einem Lödige-Mischer (Type FM 50 E 17) 6 Minuten lang mit Mixer und Chopper homogen miteinander vermischt und sodann zu dem Gemisch aus Isopropylpalmitat und Bienenwachs gegeben. Nach einer Rührzeit von 15 Minuten wird eine homogene cremeförmige Blondiermittelsuspension erhalten, die beispielsweise in handelsübliche, mit einem Innenlack versehene Aluminiumtuben abgefüllt werden kann.

**Anwendungsbeispiel a)**

25 g der Blondiermittelsuspension gemäß Beispiel 4 werden mit 25 g einer 9-prozentigen wasserstoffperoxidhaltigen Öl-in-Wasser-Emulsion der folgenden Zusammensetzung

| 18,00 g | Wasserstoffperoxid (50-prozentige wäßrige Lösung) |
|---|---|
| 2,00 g | Cetylstearylalkohol |
| 0,20 g | Lanolinalkohol |
| 0,10 g | Phosphorsäure (85-prozentig) |
| 79,70 g | Wasser |
| 100,00 g | |

in einer Schale mit einem Pinsel homogen verrührt.

Das erhaltene, breiartige Blondiermittel wird auf mittelbraunes Haar gleichmäßig aufgetragen und nach einer Einwirkungszeit von 30 Minuten bei Raumtemperatur mit warmem Wasser abgespült und getrocknet. Das so behandelte Haar ist auf einen hellblonden Farbton aufgehellt worden.

**Anwendungsbeispiel b)**

25 g der vorstehend beschriebenen Blondiermittelsuspension gemäß Beispiel 3 werden mit 37,5 g einer 6-prozentigen wäßrigen Wasserstoffperoxidlösung in einer Schale mit einem Pinsel homogen verrührt. Es ist jedoch auch möglich, die Wasserstoffperoxidlösung in einer Auftrageflasche vorzulegen und mit der Blondiermittelsuspension zum gebrauchsfertigen Blondiermittel anzuschütteln.

Das Blondiermittel wird gleichmäßig auf das aufzuhellende Haar aufgetragen und nach einer Einwirkungszeit von 40 Minuten bei Raumtemperatur mit Wasser abgespült. Sodann wird das Haar getrocknet. Der Aufhellungsgrad beträgt etwa 4 Tonstufen.

**Anwendungsbeispiel c)**

25 g der vorstehend beschriebenen Blondiermittelsuspension gemäß Beispiel 1 werden mit 75 g einer 6-prozentigen Wasserstoffperoxidemulsion in einer Auftrageflasche 10 bis 15 Sekunden geschüttelt.

Anschließend wird das Blondiermittel mittels der Auftrageflasche gleichmäßig auf das aufzuhellende Haar aufgetragen.

Nach einer Einwirkungszeit von 30 Minuten bei Raumtemperatur (20 bis 30° C) wird das Haar mit warmem Wasser gründlich ausgespült und getrocknet.

Der Aufhellungsgrad beträgt 3 Tonstufen und kann bei Verlängerung der Einwirkungszeit um 20 Minuten um 1 bis 2 Tonstufen erhöht werden.

Alle Prozentangaben stellen, soweit nicht anders angegeben, Gewichtsprozente dar.

**Patentansprüche**

1. Mittel zum Entfärben oder Blondieren von Haaren, welches unmittelbar vor der Anwendung durch Vermischen einer cremeförmigen Blondiermittelsuspension mit einem Oxidationsmittel hergestellt wird und **dadurch gekennzeichnet ist, daß** die Blondiermittelsuspension eine Mischung aus (a) mindestens einem anorganischen Persalz, (b) mindestens einem alkalisch reagierenden Salz, (c) einer Verdickerkombination, bestehend aus einem Acrylsäurepolymer und mindestens einem Polymer aus der Gruppe der Cellulosen, Alginate und Polysaccharide, (d) mindestens einem Mineralöl, (e) mindestens einem flüssigen langkettigen hydrophoben Fettsäureester, (f) mindestens einem wachsförmigen langkettigen hydrophoben Fettsäureester und/oder synthetischen Bienenwachsersatzstoff, sowie (g) gegebenenfalls Hilfsstoffen und Zusatzstoffen darstellt.

2. Mittel nach Anspruch 1, **dadurch gekennzeichnet, daß** das Persalz ausgewählt ist aus Natriumpersulfat, Kaliumpersulfat, Ammoniumpersulfat und Mischungen dieser Persulfate.

3. Mittel nach Anspruch 1 oder 2, **dadurch gekennzeichnet, daß** das Persalz in der Blondiermittelsuspension in einer Gesamtmenge von 30 bis 65 Gewichtsprozent enthalten ist.

4. Mittel nach einem der Ansprüche 1 bis 3, **dadurch gekennzeichnet, daß** das alkalisch reagierende Salz ausgewählt ist aus Natriumcarbonat, Natriumhydrogencarbonat, Magnesiumcarbonat, Ammoniumcarbonat, Ammoniumhydrogencarbonat, Natriumsilikaten und Mischungen dieser Salze ausgewählt ist.

5. Mittel nach einem der Ansprüche 1 bis 4, **dadurch gekennzeichnet, daß** das alkalisch reagierende Salz in der Blondiermittelsuspension in einer Gesamtmenge von 15 bis 45 Gewichtsprozent enthalten ist.

6. Mittel nach einem der Ansprüche 1 bis 5, **dadurch gekennzeichnet, daß** die Cellulosen, Alginate oder Polysaccharide ausgewählt sind aus der Gruppe bestehend aus Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum, Xanthan Gum und deren Mischungen.

7. Mittel nach einem der Ansprüche 1 bis 6, **dadurch gekennzeichnet, daß** die Verdickerkombination der Komponente (c) in der Blondiermittelsuspension in einer Gesamtmenge von 0,5 bis 20 Gewichtsprozent enthalten ist.

8. Mittel nach einem der Ansprüche 1 bis 7, **dadurch gekennzeichnet, daß** das Mineralöl ausgewählt ist aus Paraffin Perliquidum und Paraffin Subliquidum oder deren Mischungen.

9. Mittel nach einem der Ansprüche 1 bis 7, **dadurch gekennzeichnet, daß** der flüssige langkettige hydrophobe Fettsäureester ausgewählt ist aus Isopropylpalmitat, Octylpalmitat und Isocetylpalmitat oder deren Mischungen.

10. Mittel nach einem der Ansprüche 1 bis 7, **dadurch gekennzeichnet, daß** als wachsförmiger langkettiger hydrophober Fettsäureester Bienenwachs verwendet wird.

11. Mittel nach einem der Ansprüche 1 bis 10, **dadurch gekennzeichnet, daß** die Komponenten (d), (e) und (f) in der Blondiermittelsuspension in einer Gesamtmenge von 20 bis 35 Gewichtsprozent enthalten sind.

12. Mittel nach einem der Ansprüche 1 bis 11, **dadurch gekennzeichnet, daß** als Oxidationsmittel eine 6- bis 12-prozentige wäßrige Wasserstoffperoxidlösung oder Wasserstoffperoxidemulsion verwendet wird.

**Claims**

1. Agent for bleaching hair or dyeing it blonde, which is produced immediately before application by mixing a blonding agent suspension in cream form with an oxidizing agent and **characterized in that** the blonding agent suspension is a mixture of (a) at least one inorganic persalt, (b) at least one salt which reacts in an alkaline manner, (c) a thickener combination, consisting of an acrylic acid polymer and at least one polymer from the group of celluloses, alginates and polysaccharides, (d) at least one mineral oil, (e) at least one liquid long-chain hydrophobic fatty acid ester, (f) at least one wax-like long-chain hydrophobic fatty acid ester and/or synthetic beeswax substitute, and (g), optionally, auxiliary substances and additives.

**2.** Agent according to Claim 1, **characterized in that** the persalt is selected from sodium persulphate, potassium persulphate, ammonium persulphate and mixtures of these persulphates.

**3.** Agent according to Claim 1 or 2, **characterized in that** the persalt is contained in the blonding agent suspension in a total amount of from 30 to 65 weight per cent.

**4.** Agent according to one of Claims 1 to 3, **characterized in that** the salt which reacts in an alkaline manner is selected from sodium carbonate, sodium hydrogencarbonate, magnesium carbonate, ammonium carbonate, ammonium hydrogencarbonate, sodium silicates and mixtures of these salts.

**5.** Agent according to one of Claims 1 to 4, **characterized in that** the salt which reacts in an alkaline manner is contained in the blonding agent suspension in a total amount of from 15 to 45 weight per cent.

**6.** Agent according to one of Claims 1 to 5, **characterized in that** the celluloses, alginates or polysaccharides are selected from the group consisting of methylcelluloses, ethylcelluloses, hydroxyethylcelluloses, methylhydroxyethyl-celluloses, methylhydroxypropylcelluloses, carboxymethylcelluloses, alginic acid, sodium alginate, ammonium alginate, calcium alginate, gum arabic, guar gum, xanthan gum and mixtures thereof.

**7.** Agent according to one of Claims 1 to 6, **characterized in that** the thickener combination of component (c) is contained in the blonding agent suspension in a total amount of from 0.5 to 20 weight per cent.

**8.** Agent according to one of Claims 1 to 7, **characterized in that** the mineral oil is selected from paraffin perliquidum and paraffin subliquidum or mixtures thereof.

**9.** Agent according to one of Claims 1 to 7, **characterized in that** the liquid long-chain hydrophobic fatty acid ester is selected from isopropyl palmitate, octyl palmitate and isocetyl palmitate or mixtures thereof.

**10.** Agent according to one of Claims 1 to 7, **characterized in that** beeswax is used as wax-like long-chain hydrophobic fatty acid ester.

**11.** Agent according to one of Claims 1 to 10, **characterized in that** the components (d), (e) and (f) in the blonding agent suspension are contained in a total amount of 20 to 35 weight per cent.

**12.** Agent according to one of Claims 1 to 11, **characterized in that** a 6 to 12 per cent aqueous hydrogen peroxide solution or hydrogen peroxide emulsion is used as the oxidizing agent.

**Revendications**

**1.** Composition destinée à décolorer ou colorer en blond les cheveux, qui est préparée immédiatement avant l'emploi par mélange d'une suspension crémeuse d'agent de coloration en blond avec un oxydant et est **caractérisée en ce que** la suspension d'agent de coloration en blond représente un mélange de (a) au moins un persel inorganique, (b) au moins un sel à réaction alcaline, (c) une association d'épaississants, constituée d'un polymère d'acide acrylique et d'au moins un polymère choisi dans le groupe des celluloses, alginates et polysaccharides, (d) au moins une huile minérale, (e) au moins un ester d'acide gras hydrophobe liquide à longue chaîne, hydrophobe (f) au moins un ester d'acide gras cireux à longue chaîne, hydrophobe et/ou un substitut synthétique de cire d'abeille, ainsi que (g) éventuellement additifs et adjuvants.

**2.** Composition selon la revendication 1, **caractérisée en ce que** le persel est choisi parmi le persulfate de sodium, le persulfate de potassium, le persulfate d'ammonium et des mélanges de ces persulfates.

**3.** Composition selon la revendication 1 ou 2, **caractérisée en ce que** le persel est contenu dans la suspension d'agent de coloration en blond en une quantité totale de 30 à 65 % en poids.

**4.** Composition selon l'une quelconque des revendications 1 à 3, **caractérisée en ce que** le sel à réaction alcaline est choisi parmi le carbonate de sodium, l'hydrogénocarbonate de sodium, le carbonate de magnésium, le carbonate d'ammonium, l'hydrogénocarbonate d'ammonium, les silicates de sodium et des mélanges de ces sels.

**5.** Composition selon l'une quelconque des revendications 1 à 4, **caractérisée en ce que** le sel à réaction alcaline est contenu dans la suspension d'agent de coloration en blond en une quantité totale de 15 à 45 % en poids.

**6.** Composition selon l'une quelconque des revendications 1 à 5, **caractérisée en ce que** les celluloses, alginates et polysaccharides sont choisis dans le groupe constitué par les méthylcelluloses, les éthylcelluloses, les hydroxyéthylcelluloses, les méthylhydroxyéthylcelluloses, les méthylhydroxypropylcelluloses, les carboxyméthylcelluloses, l'acide alginique, l'alginate de sodium, l'alginate d'ammonium, l'alginate de calcium, la gomme arabique, la gomme guar, la gomme xanthane et des mélanges de ceux-ci.

**7.** Composition selon l'une quelconque des revendications 1 à 6, **caractérisée en ce que** l'association d'épaississants du composant (c) est contenue dans la suspension d'agent de coloration en blond en une quantité totale de 0,5 à 20 % en poids.

**8.** Composition selon l'une quelconque des revendications 1 à 7, **caractérisée en ce que** l'huile minérale est choisie parmi les paraffines Paraffin Perliquidum et Paraffin Subliquidum et des mélanges de celles-ci.

**9.** Composition selon l'une quelconque des revendications 1 à 7, **caractérisée en ce que** l'ester d'acide gras liquide à longue chaîne, hydrophobe, est choisi parmi le palmitate d'isopropyle, le palmitate d'octyle et le palmitate d'isocétyle et des mélanges de ceux-ci.

**10.** Composition selon l'une quelconque des revendications 1 à 7, **caractérisée en ce que** la cire d'abeille est utilisée en tant qu'ester d'acide gras cireux à longue chaîne, hydrophobe.

**11.** Composition selon l'une quelconque des revendications 1 à 10, **caractérisée en ce que** les composants (d), (e) et (f) sont contenus dans la suspension d'agent de coloration en blond en une quantité totale de 20 à 35 % en poids.

**12.** Composition selon l'une quelconque des revendications 1 à 11, **caractérisée en ce qu'**on utilise comme oxydant une solution aqueuse de peroxyde d'hydrogène ou une émulsion de peroxyde d'hydrogène à 6-12 % en poids.